# EUROPEAN PATENT APPLICATION

(11) **EP 4 332 592 A1**
(43) Date of publication of application: **06.03.2024**
(21) Application number: 23194876.1
(22) Date of filing: 01.09.2023
(51) Int. Cl.: G01R 31/3167, G06F 11/20, G06F 11/22, G06F 11/30, G06F 11/18

(54) **MULTICHANNEL APPARATUS FOR PERFORMING CHANNEL REPLACEMENT AND OPERATION METHOD OF THE SAME**

(30) Priority: 02.09.2022 KR 20220111531; 03.08.2023 KR 20230101788
(71) Applicant: Samsung Electronics Co., Ltd., Suwon-si, Gyeonggi-do 16677 (KR)
(72) Inventor: Hong, Hyeokki, 16678 Suwon-si, Gyeonggi-do (KR); Bae, Chisung, 16678 Suwon-si, Gyeonggi-do (KR); Park, Kitae, 16678 Suwon-si, Gyeonggi-do (KR)
(74) Representative: Grünecker Patent- und Rechtsanwälte PartG mbB

(57) **Abstract**

A multichannel apparatus for exchanging channels and an operating method of the multichannel apparatus are provided. The apparatus includes reception nodes configured to receive input signals of an analog domain, main signal processors configured to perform a signal processing operation on the input signals, and auxiliary signal processors configured to replace the main signal processors and perform at least a portion of the signal processing operation in response to a replacement condition being satisfied. The reception nodes, the main signal processors, and the auxiliary signal processors are implemented in a single integrated circuit (IC) package.

## Description

### 1. Field

The following description relates to an apparatus and method with electrode-based measurement.

### 2. Description of Related Art

Biosignals may be signals from plants or animals, for example, that can be continually measured and monitored. A biosignal measuring technique may measure a biosignal from a living body through a medium, such as an electrode array. A measuring device including an electrode array may be inserted into or attached to a living body, and may collect or receive a biosignal. In an example, the biosignal may include a neural signal, a brainwave signal, a muscle signal, and the like.

### SUMMARY

This Summary is provided to introduce a selection of concepts in a simplified form that is further described below in the Detailed Description. This Summary is not intended to identify key features or essential features of the claimed subject matter, nor is it intended to be used as an aid in determining the scope of the claimed subject matter.

The invention is claimed in the independent claims. Preferred embodiments are specified in the dependent claims.

In a general aspect, an apparatus includes reception nodes configured to receive input signals of an analog domain; main signal processors configured to perform a signal processing operation on the input signals; and auxiliary signal processors configured to replace the main signal processors, and perform at least a portion of the signal processing operation in response to a replacement condition being satisfied, wherein the reception nodes, the main signal processors, and the auxiliary signal processors are implemented in a single integrated circuit (IC) package.

The signal processing operation may include at least one of a digital conversion operation, a signal generation operation, a signal amplification operation, a control operation, and a clock generation operation.

Each of the main signal processors and the auxiliary signal processors may include an analog digital converter (ADC) performing a digital conversion operation on a corresponding input signal of the input signals.

The apparatus may include multiplexers (MUXs) configured to provide a signal path used by one of the auxiliary signal processors to replace one of the main signal processors.

A first main signal processor of the main signal processors may receive a first input signal of the input signals from a first reception node of the reception nodes, a first MUX of the MUXs may connect the first reception node to a first auxiliary signal processor of the auxiliary signal processors in response to the replacement condition is satisfied as a defect occurs in the first main signal processor.

The single IC package may include at least one die in which the reception nodes, the main signal processors, and the auxiliary signal processors are integrated in at least a partial form of a stack package and a system in package.

The input signals may be received through 10 or more channels.

The input signals may be received through 10,000 or more channels.

The input signals may correspond to an electrical signal.

The auxiliary signal processors may be configured to perform at least some of the signal processing operation in real-time instead of the main signal processors in response to the replacement condition being satisfied.

The main signal processors may be divided in a predetermined first number and constitute a plurality of slots, and the auxiliary signal processors nay be divided in a predetermined second number and dedicatedly and respectively allocated to the plurality of slots.

The main signal processors may be divided in a predetermined first number and constitute a plurality of slots, and the auxiliary signal processors may be allocated to the plurality of slots such that a predetermined third number of slots of the plurality of slots share at least some of the auxiliary signal processors.

The apparatus may include at least one controller configured to determine whether the main signal processors satisfy the replacement condition based on a performance test of the main signal processors.

The at least one controller may be configured to provide a test signal to the main signal processors while connection between the reception nodes and the main signal processors is open and determine whether the main signal processors satisfy the replacement condition by analyzing each processing result of the main signal processors for the test signal.

The at least one controller may be configured to, when a plurality of replacement targets satisfying the replacement condition among the main signal processors is detected, determine a replacement priority of the replacement targets based on a degree of defect due to at least one of an error and performance degradation of the replacement targets, and apply the auxiliary signal processors in order of high replacement priority.

The replacement condition may be based on a defect of the main signal processors due to at least one of an error and performance degradation of the main signal processors.

The apparatus may include an electrode array measuring the input signal through electrodes disposed on a target point.

The apparatus may include multiplexers (MUXs) connecting candidate electrodes of the electrodes to corresponding auxiliary signal processors of the auxiliary signal processors.

The main signal processors may include a first main signal processor connected to a first electrode of the electrodes, the MUXs may include a first MUX configured to selectively connect a first auxiliary signal processor of the auxiliary signal processors to first candidate electrodes comprising the first electrode, and the first MUX may be configured to connect the first electrode to the first auxiliary signal processor in response to the replacement condition being satisfied by the first main signal processor.

Before the replacement condition is satisfied, the main signal processors may be respectively connected to the electrodes.

The electrodes may include main electrodes connected to the main signal processors and auxiliary electrodes connected to the auxiliary signal processors, and the electrode array may be configured to measure the input signals using the main electrodes and the auxiliary electrodes.

The main signal processors may include a first main signal processor connected to a first main electrode of the main electrodes, the auxiliary signal processors may include a first auxiliary signal processor connected to a first auxiliary electrode of the auxiliary electrodes, and the first auxiliary signal processor may be connected to the first main electrode instead of the first main signal processor in response to the replacement condition being satisfied by the first main signal processor.

The main electrodes may be arranged in a central area of the electrode array compared to the auxiliary electrodes, and data measurement through the first auxiliary electrode may be paused in response to the replacement condition being satisfied by the first main signal processor.

The main signal processors may include first main signal processors selectively connected to each electrode of a first electrode group among the electrodes, the auxiliary signal processors may include first auxiliary signal processors selectively connected to each electrode of the first electrode group, and some of the first main signal processors and the first auxiliary signal processors selected in order from a high performance level to a low performance level may be applied to the first electrode group.

In a general aspect, an apparatus includes reception nodes configured to receive input signals of an analog domain; main signal processors configured to perform a signal processing operation on the input signals; and auxiliary signal processors configured to replace the main signal processors and perform at least a portion of the signal processing operation based on a defect of the main signal processors, wherein the reception nodes, the main signal processors, and the auxiliary signal processors are implemented in a single chip.

In a general aspect, a method includes receiving input signals of an analog domain; performing a signal processing operation on the input signals using main signal processors; and performing at least a portion of the signal processing operation using auxiliary signal processors instead of the main signal processors in response to a replacement condition being satisfied, wherein the main signal processors and the auxiliary signal processors are implemented in a single integrated circuit (IC) package or a single chip.

In a general aspect, a biologic response recording apparatus includes a measuring instrument comprising: an electrode array configured to generate input signals corresponding to data measured at target points through electrodes disposed on the target points, main signal processors configured to perform a signal processing operation comprising a digital conversion operation of corresponding input signals of the input signals, respectively, and auxiliary signal processors configured to replace the main signal processors and perform at least a portion of the signal processing operation including the digital conversion operation in response to a replacement condition being satisfied; and a memory configured to store response data output by at least some of the main signal processors and the auxiliary signal processors.

Other features and aspects will be apparent from the following detailed description, the drawings, and the claims.

### BRIEF DESCRIPTION OF DRAWINGS

FIG. 1A illustrates an example schematic structure of an example multichannel apparatus, in accordance with one or more embodiments.
FIG. 1B illustrates an example of an input device providing a signal to a multichannel apparatus, in accordance with one or more embodiments.
FIG. 2 illustrates an example configuration of a slot of an example multichannel apparatus, in accordance with one or more embodiments.
FIG. 3 illustrates an example structure of an example multichannel apparatus implemented by a chip, in accordance with one or more embodiments.
FIG. 4 illustrates an example structure of a circuit of a single slot, in accordance with one or more embodiments.
FIG. 5 is a flowchart illustrating an example test operation, in accordance with one or more embodiments.
FIG. 6 illustrates an example structure of a test circuit configured to perform a test operation, in accordance with one or more embodiments.
FIG. 7 illustrates an example relationship between components in a structure in which auxiliary signal processors are shared, in accordance with one or more embodiments.
FIG. 8 is a flowchart illustrating an example optimization operation of channel exchange, in accordance with one or more embodiments.
FIG. 9 illustrates an example operation of an electrode array including a main electrode and an auxiliary electrode, in accordance with one or more embodiments.
FIG. 10 is a flowchart illustrating an example of deriving the maximum performance by implementing auxiliary signal processors, in accordance with one or more embodiments.
FIG. 11 is a flowchart illustrating an example measuring method implementing an example electrode array, in accordance with one or more embodiments.
FIG. 12 illustrates an example configuration of an example bioreaction recording apparatus, in accordance with one or more embodiments.

Throughout the drawings and the detailed description, unless otherwise described or provided, the same or like drawing reference numerals may be understood to refer to the same elements, features, and structures. The drawings may not be to scale, and the relative size, proportions, and depiction of elements in the drawings may be exaggerated for clarity, illustration, and convenience.

### DETAILED DESCRIPTION

The following detailed description is provided to assist the reader in gaining a comprehensive understanding of the methods, apparatuses, and/or systems described herein. However, various changes, modifications, and equivalents of the methods, apparatuses, and/or systems described herein will be apparent after an understanding of the disclosure of this application. For example, the sequences of operations described herein are merely examples, and are not limited to those set forth herein, but may be changed as will be apparent after an understanding of the disclosure of this application, with the exception of operations necessarily occurring in a certain order. Also, descriptions of features that are known, after an understanding of the disclosure of this application, may be omitted for increased clarity and conciseness, noting that omissions of features and their descriptions are also not intended to be admissions of their general knowledge.

The features described herein may be embodied in different forms, and are not to be construed as being limited to the examples described herein. Rather, the examples described herein have been provided merely to illustrate some of the many possible ways of implementing the methods, apparatuses, and/or systems described herein that will be apparent after an understanding of the disclosure of this application.

Although terms such as "first," "second," and "third" may be used herein to describe various members, components, regions, layers, or sections, these members, components, regions, layers, or sections are not to be limited by these terms. Rather, these terms are only used to distinguish one member, component, region, layer, or section from another member, component, region, layer, or section. Thus, a first member, component, region, layer, or section referred to in examples described herein may also be referred to as a second member, component, region, layer, or section without departing from the teachings of the examples.

Throughout the specification, when an element, such as a layer, region, or substrate, is described as being "on," "connected to," or "coupled to" another element, it may be directly "on," "connected to," or "coupled to" the other element, or there may be one or more other elements intervening therebetween. In contrast, when an element is described as being "directly on," "directly connected to," or "directly coupled to" another element, there can be no other elements intervening therebetween. Likewise, expressions, for example, "between" and "immediately between" and "adjacent to" and "immediately adjacent to" may also be construed as described in the foregoing.

The terminology used herein is for the purpose of describing particular examples only, and is not to be used to limit the disclosure. As used herein, the singular forms "a," "an," and "the" are intended to include the plural forms as well, unless the context clearly indicates otherwise. As used herein, the term "and/or" includes any one and any combination of any two or more of the associated listed items. As used herein, the terms "include," "comprise," and "have" specify the presence of stated features, numbers, operations, elements, components, and/or combinations thereof, but do not preclude the presence or addition of one or more other features, numbers, operations, elements, components, and/or combinations thereof. The use of the term "may" herein with respect to an example or embodiment (for example, as to what an example or embodiment may include or implement) means that at least one example or embodiment exists where such a feature is included or implemented, while all examples are not limited thereto.

As used herein, for phrases "at least one of A and B", "at least one of A, B, or C," and the like, each list may include any one of the listed items in the corresponding one of the phrases, or all possible combinations thereof.

Unless otherwise defined, all terms including technical and scientific terms, used herein have the same meaning as commonly understood by one of ordinary skill in the art to which this disclosure pertains consistent with and after an understanding of the present disclosure. Terms, such as those defined in commonly used dictionaries, are to be interpreted as having a meaning that is consistent with their meaning in the context of the relevant art and the present disclosure, and are not to be interpreted in an idealized or overly formal sense unless expressly so defined herein.

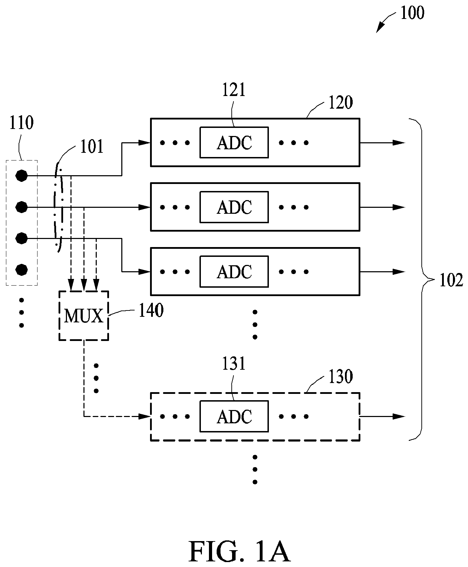

FIG. 1A illustrates an example schematic structure of an example multichannel apparatus, in accordance with one or more embodiments. Referring to FIG. 1A, a multichannel apparatus 100 may include reception nodes 110. The multichannel apparatus 100 may receive input signals 111 through the reception nodes 110 and may perform signal processing on the input signals 111. The reception nodes 110 may respectively correspond to points on various types of signal transmission paths through which the input signals 111 are received as well as a specific structure, such as a terminal receiving the input signals 111.

The input signals 111 may be received through multiple channels. For example, the input signals 111 may be received through 10 or more channels or 10,000 or more channels. Input signals 101 may be electrical signals. For example, the input signals 101 may be transmitted by various input devices using multichannel, such as a signal measuring instrument, a sensor (e.g., a pressure mapping sensor, an image sensor, a lidar sensor), a crossbar array, and an Internet of Things (lOT) device. The input signals 101 may correspond to light, temperature, velocity, pressure, and the like. In this case, the input signals 101 may be converted into electrical signals by a main signal processors and/or auxiliary signal processors of the multichannel apparatus 100.

The multichannel apparatus 100 may include a plurality of main signal processors, such as a main signal processor 120. In an example, the main signal processor 120 may correspond to an analog front end (AFE). Each of the main signal processors may perform signal processing operations on a corresponding input signal among the input signals 101. Each of the main signal processors may configure each signal transmission channel. Each corresponding input signal may have a corresponding relationship with each of the main signal processors. For example, when the input signals 101 include a first input signal, a second input signal, and a third input signal, and the plurality of main signal processors include a first main signal processor configured to receive the first input signal, a second main signal processor configured to receive the second input signal, a third main signal processor configured to receive the third input signal, the first main signal processor and the first input signal, the second main signal processor and the second input signal, and the third main signal processor and the third input signal may have corresponding relationships, respectively.

According to one embodiment, the input signals 101 may correspond to an analog signal. According to one embodiment, signal processing may include a digital conversion operation. In implementing the digital conversion operation, an input signal may be converted from an analog domain to a digital domain. Each of the main signal processors may include an analog to digital converter (ADC), and may perform a digital conversion operation on the corresponding input signal using the ADC.

According to one embodiment, the reception nodes, the main signal processors, and the auxiliary signal processors may be implemented in a single integrated circuit (IC) package. The single IC package may include at least one die in which the reception nodes, the main signal processors, and the auxiliary signal processors are integrated in at least a partial form of a stack package and a system in package (SiP). For example, the stack package may be based on at least one of package stack or chip stack. The chip stack may be based on at least one of wire bonding and through silicon via (TSV). For example, the SiP may be based on an interposer.

According to one embodiment, the reception nodes, the main signal processors, and the auxiliary signal processors may be implemented in a single chip. The reception nodes, the main signal processors, and the auxiliary signal processors may be disposed on the same die that is the single chip. When the reception nodes, the main signal processors, and the auxiliary signal processors are implemented in a single IC package or a single chip and a defect occurs in the main signal processors, the auxiliary signal processors may effectively replace the main signal processors. Replacing the main signal processors with the auxiliary signal processors may mean that the auxiliary signal processors may be able to sufficiently perform functions of the main signal processors as needed. When the main signal processors and the auxiliary signal processors are implemented in different IC packages or different chips, the auxiliary signal processors may not replace the main signal processors.

According to one embodiment, signal processing may include various operations that are performed through various components. For example, signal processing may include a signal generation operation using a signal generator (e.g., a current generator, a voltage generator, etc.), a signal amplification operation using an amplifier, a control operation using a controller, and a clock generation operation using a clock generator. The signal generator and the amplifier may be included in the analog domain, and the controller and the clock generator may be included in the digital domain. The analog domain and the digital domain may be distinguished by the ADC.

The multichannel apparatus 100 may include a plurality of auxiliary signal processors, such as an auxiliary signal processor 130. Before a replacement condition is satisfied, the main signal processors are respectively connected to the reception nodes 110. In response to the replacement condition of the main signal processors 120 being satisfied, the auxiliary signal processor 130 may at least partially perform signal processing of the main signal processor 120 instead of the main signal processor 120. When the replacement condition is satisfied, the auxiliary signal processors may at least partially perform signal processing instead of the main signal processors in real-time. For example, the replacement condition may include an occurrence of a defect in at least a portion of signal processing of the main signal processor 120. For example, the defect may include an error or a performance degradation. The replacement condition may be based on a defect of the main signal processors due to at least one of an error and a performance degradation of the main signal processors. Performance degradation may be regarded as a more severe defect than the occurrence of an error. An error may refer to a state in which signal processing is barely or completely not performed and performance degradation may refer to a state in which signal processing is performed at a low level. For example, when an error occurs in the ADC of the main signal processor 120, or the performance of the ADC of the main signal processor 120 degrades, the occurrence of the error or the occurrence of the performance degradation of the ADC in the main signal processor 120 may satisfy the replacement condition. In this example, the auxiliary signal processor 130 may at least partially replace signal processing operations of the main signal processor 120. The plurality of auxiliary signal processors may include ADCs, respectively, and may perform a digital conversion operation of a corresponding input signal using the ADC instead of the main signal processors. The replacement of the main signal processor 120 by the auxiliary signal processor 130 may be referred to as channel exchange.

The multichannel apparatus 100 may include a plurality of multiplexers (MUX), such as a MUX 140. In a non-limited example, the number of MUXs 140 may be the same as the number of auxiliary signal processors 130. However, this is only an example, and the number of MUXs 140 may be different from the number of auxiliary signal processors 130. A MUX may provide a signal path that is used by an auxiliary signal processor 130 to replace a main signal processor 120. For example, a first main signal processor of the main signal processors may receive a first input signal of input signals from a first reception node of the reception nodes and a first MUX of the MUXs may connect the first reception node to a first auxiliary signal processor of the auxiliary signal processors as a defect occurs in the first main signal processor and the replacement condition is satisfied.

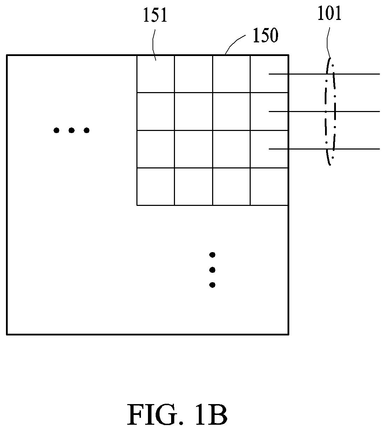

The size of the MUX 140 may depend on the number of reception nodes covered by the MUX 140 and/or the number of main signal processors 120. For example, as illustrated in FIG. 1, when the MUX 140 covers or controls three electrodes and/or three signal processors, the MUX 140 may have a size corresponding to the number of covered electrodes and/or signal processors.

The electrodes that are covered or controlled by the MUX 140 may be referred to as candidate nodes (e.g., candidate electrodes). In a non-limited example, each MUX 140 may correspond to each auxiliary signal processor 130. Each MUX 140 may selectively connect each candidate node to a corresponding auxiliary signal processor 130. Selective connection may represent a state that may open or close through a circuit control (e.g., switching). For example, when the main signal processor 120 is connected to a first node, the MUX 140 may selectively connect candidate nodes including the first node to the auxiliary signal processor 130. In this example, in response to the main signal processor 120 that satisfies the replacement condition, the MUX 140 may connect the first electrode 111 to the auxiliary signal processor 130.

The number of connectable input signals of the auxiliary signal processor 130 may vary based on the size of the MUX 140, and by adjusting the size of the MUX 140, and through this process, a coverable range of the auxiliary signal processor 130 and a complexity of the MUX 140 may be determined. The main signal processors 120 and the auxiliary signal processors 130 may produce output signals 102 based on input signals 101. The output signal(s) 102 may include a measurement result of a target point. For example, the output signal(s) 102 may include a bioreaction or a biological (or biosignal), such as a neural signal, a brainwave signal, a muscle signal, a temperature, and a location. In an example, the output signal may refer to a change in current produced by the sum of an electrical potential difference across a specialized tissue, organ or cell system of a living organism.

According to one embodiment, electrodes of the reception nodes 110 may form high-density multi-channels. In a non-limited example, the number of channels may be more than 10K. Additionally, the reception nodes 110 may be implemented by a single chip (e.g., a biochip). In this example, when producing a high-density multi-channel single chip, it may be difficult to apply a process of selecting a good die. This characteristic may significantly decrease a production yield rate. Additionally, electrodes in the reception nodes 110 may need to be utilized as much as possible. However, when the multichannel apparatus 100 is inserted into a body, it may be difficult to remove the multichannel apparatus 100 from the body and repair the multichannel apparatus 100 even though a problem has occurred in the multichannel apparatus 100. In addition, in the case of data that requires real-time processing, such as autonomous driving, instance maintenance may be required without maintenance by human.

Even if a problem occurs due to a defect in some channels of the multichannel apparatus 100 when the multichannel apparatus 100 is implemented, the problems related to the defective channels may be solved by the implementation of an auxiliary signal processor, and thus, the production yield rate of the multichannel apparatus 100 may improve and the usage period of the multichannel apparatus 100 may be extended. Additionally, the auxiliary signal processor 130 may increase, maximize and/or optimize the performance of the multichannel apparatus 100.

FIG. 1B illustrates an example of an input device providing a signal to a multichannel apparatus, in accordance with one or more embodiments. Referring to FIG. 1B, an input device 150 may transmit the input signal 101 to the multichannel apparatus. According to one embodiment, the input device 150 may include an electrode array. For example, the electrode array may be a multichannel microelectrode array (MEA). Hereinafter, an electrode array may be provided as an example of the input device 150. However, the input device 150 is not limited to the electrode array.

The electrode array may include a plurality of electrodes, such as an electrode 151. The plurality of electrodes may be arranged or disposed on target points, and input signals 101 of an analog domain may be measured through the plurality of electrodes. The input signals 101 may be transmitted to the multichannel apparatus through the reception nodes. As an example, the target points may be a living body including some or all parts of various living organisms, such as, but not limited to, a human body, an animal, a plant, and the like. In an example, the targets may be neurons or nerve cells. The electrodes may include a metal material and may contact a target point. The input signal may be measured by each electrode and the number of input signals 101 may be the same as the number of electrodes. Before the replacement condition of the main signal processors is satisfied, the main signal processors may be connected to electrodes of the electrode array, respectively.

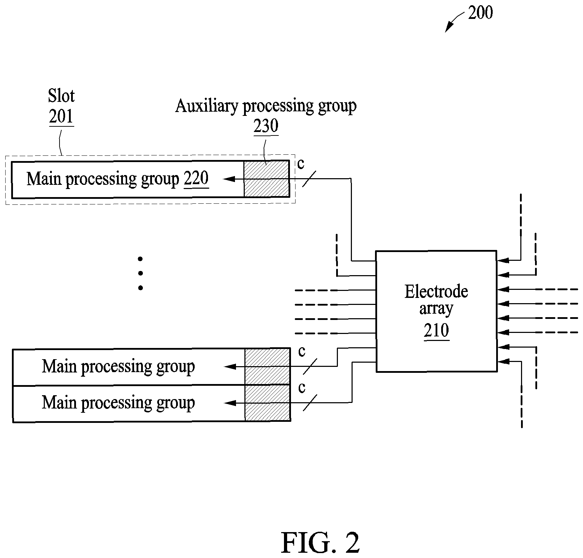

FIG. 2 is a diagram illustrating an example configuration of a slot of an example multichannel apparatus, in accordance with one or more embodiments.

Referring to FIG. 2, an example multichannel apparatus 200 may include an electrode array 210, main processing groups, such as a main processing group 220, and auxiliary processing groups, such as an auxiliary processing group 230. Each main processing group may include a plurality of main signal processors, and each auxiliary processing group 230 may include a plurality of auxiliary signal processors.

In an example, each main processing group may configure one slot (e.g., a slot 201). In an example, the main signal processors may be divided into a predetermined first number, and may configure a plurality of slots and the auxiliary signal processors may be divided into a predetermined second number and may be allocated to each of the plurality of slots.

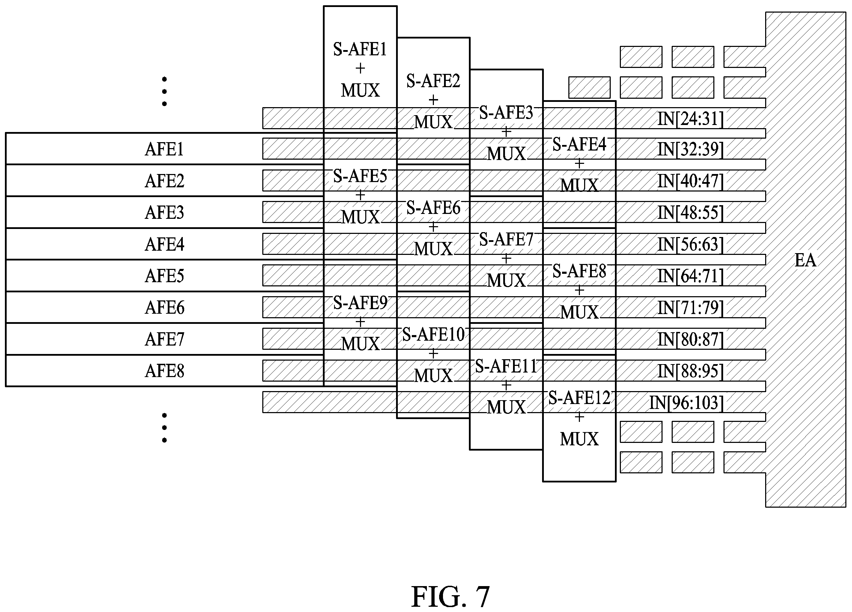

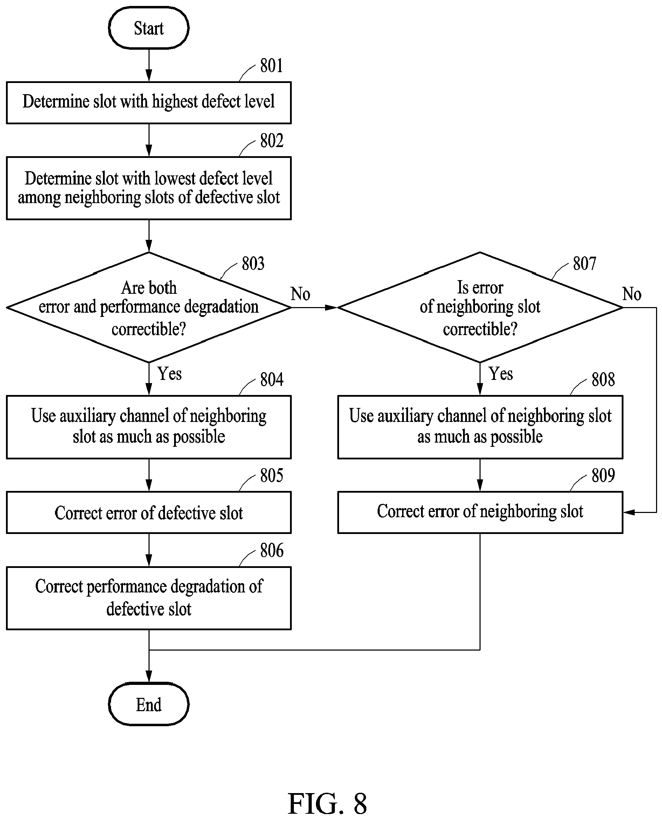

Referring to FIG. 2, c may denote the predetermined first number of main signal processors. For example, the predetermined first number of main signal processors may be 32 and the second number may be 4. In this example, 32 main signal processors may configure one slot and for each slot, 4 auxiliary signal processors may be allocated. In this example, each slot may process 32 channels. Referring to an example illustrated in FIG. 2, the second number of auxiliary signal processors may be dedicatedly allocated to each slot. However, as illustrated in FIGS. 7 and 8, main signal processors in different slots may share any auxiliary signal processors.

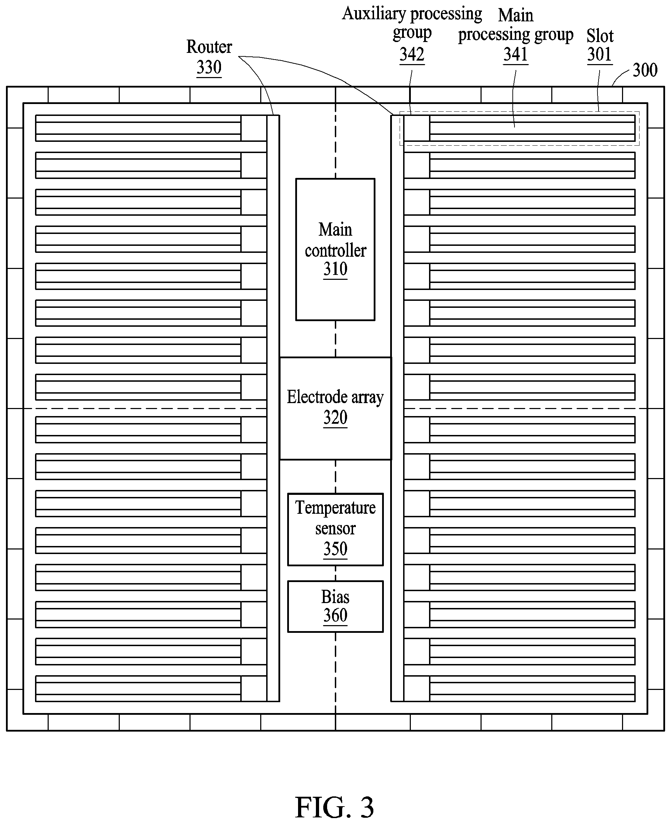

FIG. 3 is a diagram illustrating an example structure an example multichannel apparatus implemented by a chip, in accordance with one or more embodiments.

Referring to FIG. 3, an example multichannel apparatus 300 may include a main controller 310, an electrode array 320, a router 330, main processing groups, such as a main processing group 341, auxiliary processing groups, such as an auxiliary processing group 342, a temperature sensor 350, and a bias circuit 360. Each main processing group may constitute one slot (e.g., a slot 301).. The main controller 310 may be configured to control and calibrate an overall measurement operation. For example, the main controller 310 may perform a command to run a test to each channel, may perform a command channel exchange based on a test result, may collect an output signal of each channel, and may generate a final output signal. A test operation may be performed under control by the main controller 310, under control by other controllers (e.g., a digital circuit block), or cooperation of the main controller with other controllers. The router 330 may be configured to perform communication by transmitting an input signal, transmitting an output signal, and based on a command. The temperature sensor 350 may be configured to sense a temperature from an input signal and the bias circuit 360 may be configured to control a bias of an analog signal. The structure illustrated in FIG. 3 is merely an example, and the multichannel apparatus 300 may be implemented in a chip having a different structure from FIG. 3. For example, instead of the electrode array 320, reception nodes or other input devices may be disposed.

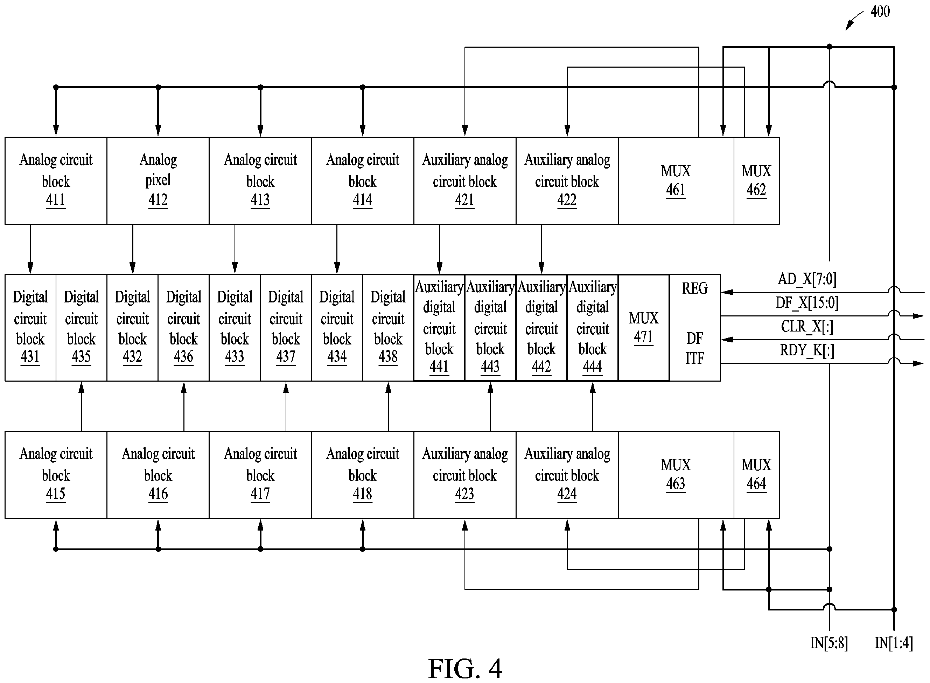

FIG. 4 is a diagram illustrating an example structure of a circuit of a single slot, in accordance with one or more embodiments. Referring to FIG. 4, a slot 400 may include analog circuit blocks 411 to 418, auxiliary analog circuit blocks 421 to 424, digital circuit blocks 431 to 438, auxiliary circuit blocks digital circuit blocks441 to 444, MUXs 461 to 464 and 471, a register (REG), a digital filter (DF), and an interface (ITF). One analog circuit block (e.g., the analog circuit block 411) and one digital circuit block (e.g., the digital circuit block 431) may constitute one pixel. The pixel may refer to a circuit unit processing a predetermined number of channels (e.g., a single channel). The pixel may correspond to a main signal processor and/or an auxiliary signal processor. Although FIG. 4 illustrates an example that the slot 400 includes 8 main channels and 4 auxiliary channels, this is only an example, and the number of main channels and/or auxiliary channels may vary.

Corresponding pairs of the analog circuit blocks 411 to 418 and the digital circuit blocks 431 to 438 may correspond to the main signal processors and/or the main channels. The corresponding pairs of the auxiliary analog circuit blocks 421 to 424 and the auxiliary digital circuit blocks 441 to 444 may correspond to the auxiliary signal processors and/or the auxiliary channels. Each of the analog circuit blocks 411 to 418 and the auxiliary analog circuit blocks 421 to 424 may include at least one of an ADC, a signal generator (e.g., a current generator and a voltage generator), and an amplifier. Each of the digital circuit blocks 431 to 438 and the auxiliary digital circuit blocks 441 to 444 may include at least one of a controller and a clock generator.

When at least some of the main signal processors and/or the main channels satisfy a replacement condition, the at least some of the main signal processors and/or the main channels may be replaced by the auxiliary signal processors and/or the auxiliary channels. For channel exchange, the MUXs 461 to 464 may provide an input signal IN of a replacement target to at least some of the auxiliary signal processors and/or the auxiliary channels.

The main controller may transmit a request signal AD_X to the slot 400 by specifying a specific channel. The MUX 471 may select an output signal DF_X of the specific channel based on the request signal AD_X from among output signals of a plurality of channels. The main controller may notify with a clear signal CLR_X whether the output signal DF_X is received. A ready signal RDY_K may be used to determine whether the main controller is able to receive the output signal DF_X. The input signal IN may be provided to the analog circuit blocks 411 to 418 and the MUXs 461 to 464 from an electrode array.

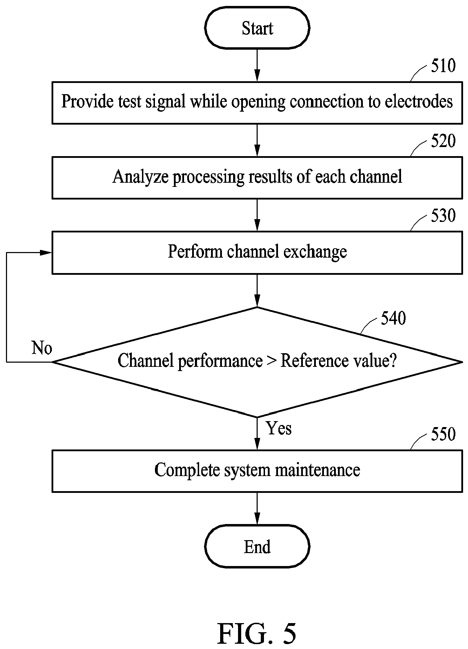

FIG. 5 is a flowchart illustrating an example test operation, in accordance with one or more embodiments. One or more blocks of FIG. 5, and combinations of the blocks, can be implemented by special purpose hardware-based computer that perform the specified functions, or special purpose hardware configured by execution of computer instructions by one or more processors. In addition to the description of FIG. 5 below, the descriptions of FIGS. 1-4 are also applicable to FIG. 5, and are incorporated herein by reference. Thus, the above description may not be repeated here.

Referring to FIG. 5, in operation 510, a test signal may be provided while a connection to electrodes is open. Since an external input signal may enters through the electrodes, as the connection to the electrodes opens, an independent test for each channel may be performed while an external connection is blocked. The test signal may have a known waveform (e.g., a triangular waveform, a sine waveform, and the like). Each channel may correspond to each main signal processor and/or each auxiliary signal processor.

In operation 520, a processing result of each channel may be analyzed. A result signal of each channel may be determined as the test signal passes through each channel. The processing result may be determined based on a difference between the test signal and the result signal. When there is a channel with a large difference, the channel may be determined to be defective. A defect may be determined, as only examples, to be an error or performance degradation based on the level of the difference. It may be determined that the defective channel satisfies a replacement condition.

In operation 530, channel exchange may be performed. The defective channel may be replaced with a different channel, for example, an auxiliary channel. For example, when a main channel of a main signal processor is defective, the main channel may be replaced by an auxiliary channel of an auxiliary signal processor. When replacement targets satisfying the replacement condition are detected from the main signal processors, a replacement priority of the replacement targets may be determined based on the level of defects of the replacement targets, and the auxiliary signal processors may be applied in order from a highest priority to a lowest priority. For example, an error channel may have a higher priority than a performance degraded channel.

In operation 540, the channel performance may be compared to a reference value and operation 530 may be repeated until the channel performance exceeds the reference value. Channel exchange for various channels during iterations may be performed. When the channel performance exceeds the reference value, in operation 550, system maintenance may be completed.

An execution time of operations 510 to 550 may include at least one of a time point of initial booting of the system, a time point based on a preset period, and a time point based on an aperiodic request. Operations 510 to 550 may be performed by a configuration of each channel under control by the main controller of the multichannel apparatus.

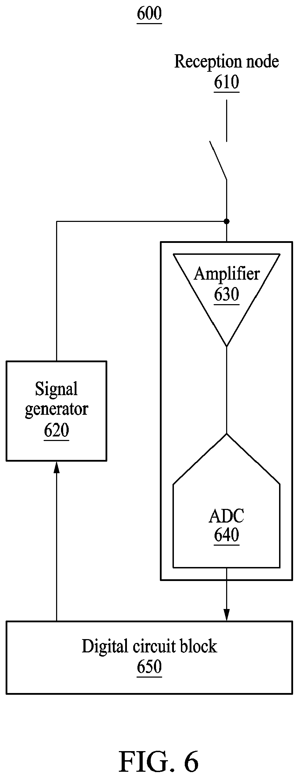

FIG. 6 is a block diagram illustrating an example structure of a test circuit configured to perform a test operation, in accordance with one or more embodiments.

Referring to FIG. 6, a test circuit 600 may include a digital circuit block 650, a signal generator 620, an amplifier 630, and an ADC 640. The test circuit 600 may correspond to at least a portion of each channel. At least a portion (e.g., the amplifier 630, the ADC 640, and a digital circuit block) of the test circuit 600 may correspond to at least a portion of a main signal processor and/or an auxiliary signal processor. The other portion (e.g., the signal generator 620) of the test circuit 600 may correspond to at least a portion of the main signal processor and/or the auxiliary signal processor, or may be implemented on the outside of the main signal processor and/or the auxiliary signal processor.

The signal generator 620 may generate a test signal based on control of the digital circuit block 650, and the test signal may be provided to a channel while an electrode 610 is blocked from the channel. The channel may include the amplifier 630 and/or the ADC 640. As the test signal passes through a channel, a result signal may be determined. The digital circuit block 650 may perform channel exchange based on the result signal.

Although an example in which a test operation is performed under control by the digital circuit 650, the test operation may be performed under control by a main controller (e.g., the main controller 310 of FIG. 3), under control by the digital circuit block 650 corresponding to another controller, or cooperation of the main controller with the digital circuit block 650.

FIG. 7 is a diagram illustrating an example relationship between components in a structure in which auxiliary signal processors are shared, in accordance with one or more embodiments.

FIG. 7 illustrates a connection relationship between an electrode array EA, an input signal IN, auxiliary (or sub) AFEs S-AFE 1 to S-AFE 12, and main AFE groups AFE 1 to AFE 8. An AFE may correspond to a main processing group and S-AFE may correspond to an auxiliary signal processor. For ease of description, each auxiliary signal processor is displayed by being merged with a corresponding MUX.

Each main AFE group may include a first number of main signal processors and a third number of main AFE groups may share one auxiliary signal processor, where the first number and the third number are predetermined. FIG. 7 illustrates an example that the first number is 8 and the third number is 4. In this example, main AFE groups that are close to each other (e.g., neighboring main AFE groups) among the main AFE groups, may share auxiliary signal processors. For example, the main AFE groups AFE 1 to AFE 4 may share the auxiliary AFE S-AFE 5.

The auxiliary AFEs may be distributed to the main AFE groups in a predetermined pattern. The main AFE groups, the auxiliary AFE, and the third number may be defined by AFE i, S-AFE j, and k, respectively. In this example, S-AFE i+1, i+2, ..., i+k may be allocated to AFE i. For example, when k is 4 as illustrated in FIG. 7, S-AFE 2, 3, 4, and 5 may be allocated to AFE 1 and S-AFE 3, 4, 5, and 6 may be allocated to AFE 2.

Referring to FIG. 7, [c1:c2] may specify channels of c1 to c2. For example, the main AFE group AFE 1 may include 8 main signal processors and may receive input signals IN[32:39] of the 32th channel to the 39th channel. The main AFE groups AFE 1 to AFE 4 may be allocated to the auxiliary AFE S-AFE 5, and thus, the auxiliary AFE S-AFE 5 and the corresponding MUX may cover input signals IN[32:63].

FIG. 8 is a flowchart illustrating an example of an optimization operation of channel exchange, in accordance with one or more embodiments. One or more blocks of FIG. 8, and combinations of the blocks, can be implemented by special purpose hardware-based computer that perform the specified functions, or special purpose hardware configured by execution of computer instructions by one or more processors. In addition to the description of FIG. 8 below, the descriptions of FIGS. 1-7 are also applicable to FIG. 8, and are incorporated herein by reference. Thus, the above description may not be repeated herein. When defects simultaneously occur in different slots that share a predetermined auxiliary signal processor, a problem that which main processing group uses the auxiliary signal processor may occur. FIG. 8 may provide a method of optimizing allocation of auxiliary signal processors. According to one embodiment, operations 801 to 809 may be performed as operation 530 of FIG. 5.

Referring to FIG. 8, in operation 801, a multichannel apparatus may determine a slot with the highest level of a defect. The defect may include an error and performance degradation. A defect level may be determined based on the number of error channels in each slot. When the number of error channels is the same, the defect level of each slot may be determined by additionally considering the number of performance degraded channels. A slot with the highest defect level may be referred to as a defective slot.

In operation 802, the multichannel apparatus may determine a slot with the lowest defect level among neighboring slots of the defective slot. A neighboring slot may refer to a slot arranged adjacent to a defective slot or a slot that shares an auxiliary signal processor with the defective slot. When there are many channels that may have to be exchanged in the defective slot, a plurality of neighboring slots may be selected. For example, when a defective slot performs channel exchange through a sub channel that is shared with a first neighboring slot and a second neighboring slot, and the first neighboring slot has a defect and the second neighboring slot does not have a defect, the defective slot may perform channel exchange through the sub channel shared with the second neighboring slot.

In operation 803, the multichannel apparatus may determine whether the error and performance degradation are correctable. The multichannel apparatus may determine whether there are sufficient auxiliary signal processors to correct errors and performance degradation of the defective slot and the neighboring slots by considering conditions of the neighboring slots.

In operation 804, the multichannel apparatus may determine to use the sub channel of the neighboring slot as much as possible, in operation 805, the multichannel apparatus may correct errors of the defective slot and the neighboring slot, and in operation 806, the multichannel apparatus may correct performance degradation of the neighboring slot and the defective slot. Error correction may take precedence over defective slot correction.

When correction of both errors and performance degradation is determined to be difficult in operation 803, operation 807 may be performed. In operation 807, the multichannel apparatus may determine whether error correction of the neighboring slot is available. In operation 808, the multichannel apparatus may determine whether to use the sub channel of the neighboring slot as much as possible. In operation 809, the multichannel apparatus may correct errors of the defective slot and the neighboring slot.

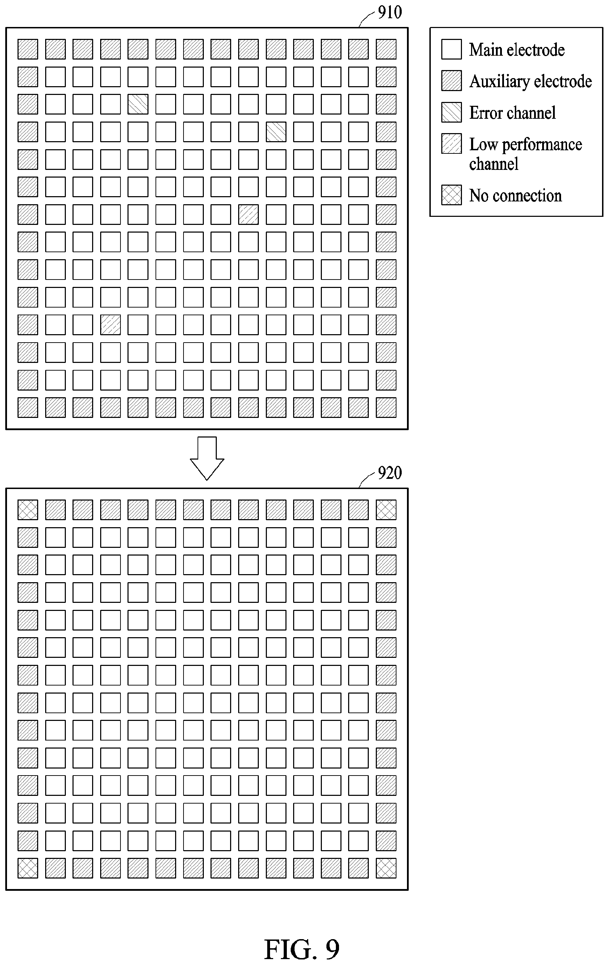

FIG. 9 is a diagram illustrating an example operation of an electrode array including a main electrode and an auxiliary electrode, in accordance with one or more embodiments.

Referring to FIG. 9, an electrode array may be controlled to switch from a first operation state 910 to a second operation state 920 based on a channel exchange operation. The electrode array may include main electrodes and auxiliary electrodes. The main electrodes may be arranged in the central area of the electrode array compared to the auxiliary electrodes. The electrode array may measure input signals by using the main electrodes and the auxiliary electrodes. According to an example of FIG. 9, even if channel exchange of a main channel is not necessary, the auxiliary channel may operate as an independent measuring channel.

In the first operation state 910 of the electrode array, an error channel and a performance degraded channel (hereinafter, referred to as a low performance channel) among the main electrodes may be detected. The main electrodes may correspond to main channels and may be connected to main signal processors. The auxiliary electrodes may correspond to auxiliary channels and may be connected to auxiliary signal processors. In the second operation state 920, channel exchange for the main electrode of the error channel and the low performance channel may be performed. Based on channel exchange, an auxiliary channel of the auxiliary electrode may be applied to the main electrode of the error channel and the low performance channel. The previous auxiliary electrode to which the auxiliary channel used for channel exchange is connected may be in a connection lost state. For example, a first main signal processor may be connected to a first main electrode among the main electrodes and a first auxiliary signal processor may be connected to a first auxiliary electrode among the auxiliary electrodes. As the first main signal processor satisfies a replacement condition, the first auxiliary signal processor may replace the first main signal processor and may be connected to the first main electrode.

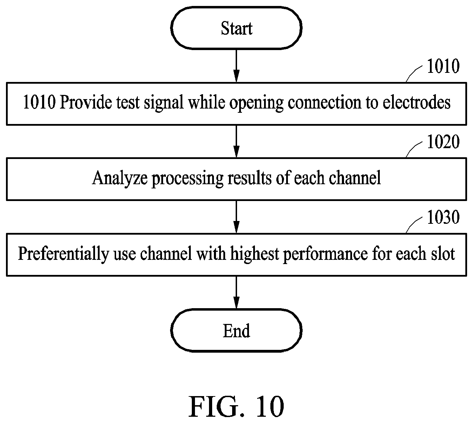

FIG. 10 is a flowchart illustrating an example of deriving the maximum performance by implementing auxiliary signal processors, in accordance with one or more embodiments. One or more blocks of FIG. 10, and combinations of the blocks, can be implemented by special purpose hardware-based computer that perform the specified functions, or special purpose hardware configured by execution of computer instructions by one or more processors. In addition to the description of FIG. 10 below, the descriptions of FIGS. 1-9 are also applicable to FIG. 10, and are incorporated herein by reference. Thus, the above description may not be repeated herein.

Referring to FIG. 10, each auxiliary signal processor may be in a state where they are selectively connectable to all electrodes in a slot through a corresponding MUX. In this example, main signal processors in the slot and the auxiliary signal processors may compete with each other to connect to an electrode, and a main signal processor or an auxiliary signal processor with the highest performance for the electrode may be connected to the electrode. For example, when a slot includes first main signal processors selectively connected to each electrode of a first electrode group among electrodes in an electrode array, and first auxiliary signal processors selectively connected to each electrode of the first electrode group, some of the first main signal processors and the first auxiliary signal processors selected in order of high performance may be applied to the first electrode group.

Referring to FIG. 10, in operation 1010, a test signal may be provided while connection to electrodes is open and in operation 1020, a processing result of each channel may be analyzed. The descriptions provided with reference to FIGS. 5 and 6 may apply to operations 1010 and 1020. In operation 1030, a channel with the highest performance for each slot may be preferentially used. In an electrode of a slot, when a main channel shows higher performance than an auxiliary channel, a measurement operation may be performed through a main signal processor of the main channel and in another electrode, when an auxiliary channel shows higher performance than a main channel, a measurement operation may be performed through an auxiliary signal processor of the auxiliary channel. According to an example of FIG. 10, the best performance of each slot may be derived through an auxiliary channel.

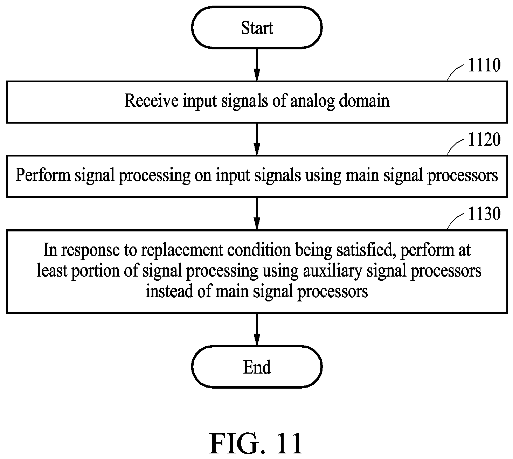

FIG. 11 is a flowchart illustrating an example of a measuring method using an electrode array, in accordance with one or more embodiments. One or more blocks of FIG. 11, and combinations of the blocks, can be implemented by special purpose hardware-based computer that perform the specified functions, or special purpose hardware configured by execution of computer instructions by one or more processors. In addition to the description of FIG. 11 below, the descriptions of FIGS. 1-10 are also applicable to FIG. 11, and are incorporated herein by reference. Thus, the above description may not be repeated herein.

Referring to FIG. 11, in operation 1110, a multichannel apparatus may receive input signals of an analog domain. In operation 1120, the multichannel apparatus may perform signal processing on the input signals using main signal processors, and in operation 1130, in response to a replacement condition being satisfied, the multichannel apparatus may perform at least a portion of signal processing using auxiliary signal processors instead of the main signal processors.

Additionally, the descriptions provided with reference to FIGS. 1 to 10 and FIG. 12 may apply to the measuring method of FIG. 11.

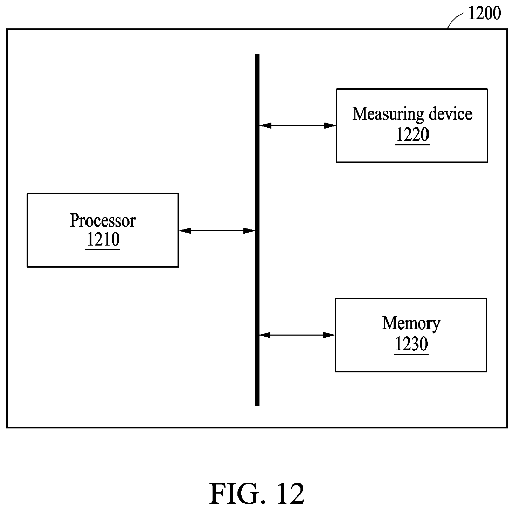

FIG. 12 is a block diagram illustrating an example of a configuration of an example apparatus, e.g., as or including, a bioreaction recording apparatus, in accordance with one or more embodiments.

Referring to FIG. 12, a bioreaction recording apparatus 1200 may include one or more processors 1210, a measuring device 1220, and a memory 1230. The memory 1230 may be connected to the one or more processors 1210 and the measuring device 1220, and may store instructions executable by the one or more processors 1210, data to be computed by the one or more processors 1210, or data processed by the one or more processors 1210. The memory 1230 may include a non-transitory computer-readable medium, for example, high-speed random-access memory (RAM), and/or a nonvolatile computer-readable storage medium (e.g., one or more disk storage devices, flash memory devices, or other nonvolatile solid state memory devices). The measuring device 1220 may measure a bioreaction and the memory 1230 may store the measured bioreaction. The one or more processors 1210 may control the measuring device 1220 and the memory 1230.

The measuring device 1220 may correspond to a multichannel apparatus. The measuring device 1220 may perform the operations of FIGS. 1 to 11. For example, the measuring device 1220 may include an electrode array configured to measure input signals of an analog domain through electrodes arranged in a target point, main signal processors configured to perform signal processing including a digital conversion operation of corresponding input signals of the input signals, respectively, and in response to a replacement condition being satisfied, auxiliary signal processors configured to replace the main signal processors and perform at least some of the signal processing comprising the digital conversion operation.

The memory 1230 may store response data output by at least some of the main signal processors and the auxiliary signal processors.

The measuring device 1220 may further include MUXs that selectively connect candidate electrodes of the electrodes to corresponding auxiliary signal processors of the auxiliary signal processors.

The main signal processors may include a first main signal processor connected to a first electrode of the electrodes, the MUXs may include a first MUX configured to selectively connect a first auxiliary signal processor of the auxiliary signal processors to first candidate electrodes including the first electrode, and in response to the replacement condition being satisfied by the first main signal processor, the first MUX may connect the first electrode to the first auxiliary signal processor.

The main signal processors may be divided into a predetermined first number, and may configure a plurality of slots, and the auxiliary signal processors may be divided into a predetermined second number, and may be dedicatedly allocated to each of the plurality of slots.

The main signal processors may be divided into a predetermined first number and may configure a plurality of slots, and the auxiliary signal processors may be allocated to the plurality of slots such that a predetermined third number of slots of the plurality of slots share at least a portion of the auxiliary signal processors.

The measuring device 1220 may further include a controller configured to determine whether the main signal processors satisfy the replacement condition based on a performance test of the main signal processors.

The controller may be configured to, while connection between the electrodes and the main signal processors is open, provide a test signal to each of the main signal processors, analyze a processing result of each of the main signal processors in response to the test signal, and determine whether the main signal processors satisfy the replacement condition.

The controller may be configured to, when a plurality of replacement targets satisfying the replacement condition is detected from the main signal processors, determine a replacement priority of the replacement targets based on a defect level of the replacement targets and apply the auxiliary signal processor in order from a highest priority down.to a lowest priority

The electrodes may include main electrodes connected to the main signal processors, and auxiliary electrodes connected to the auxiliary signal processors, and the electrode array may be configured to measure the input signals using the main electrodes and the auxiliary electrodes.

The main signal processors may include a first main signal processor connected to a first main electrode among the main electrodes, the auxiliary signal processors may include a first auxiliary signal processor connected to a first auxiliary electrode among the auxiliary electrodes, and in response to the replacement condition being satisfied by the first main signal processor, the first auxiliary signal processor may replace the first main signal processor, and may be connected to the first main electrode.

The main electrodes may be arranged in a central area of the electrode array compared to the auxiliary electrodes, and in response to the replacement condition being satisfied by the first main signal processor, data measurement through the first auxiliary electrode may be paused.

The main signal processors may include first main signal processors selectively connected to each electrode of a first electrode group among the electrodes, the auxiliary signal processors may include first auxiliary signal processors selectively connected to each electrode of the first electrode group, and some of the first main signal processors and the first auxiliary signal processors selected in order from high performance may be applied to the first electrode group.

In an example, the electrode array may be a multi-channel MEA.

Additionally, the descriptions provided with reference to FIGS. 1 to 11 may apply to the bioreaction recording apparatus 1200.

The main signal processors 120, auxiliary signal processors 130, MUX 140, controller 310, temperature sensor 350, signal generator 620, amplifier 630, ADC 640, digital circuit block 650, one or more processors 1210, measuring device 1220, memory 1230, and other devices, and other components described herein are implemented as, and by, hardware components. Examples of hardware components that may be used to perform the operations described in this application where appropriate include controllers, sensors, generators, drivers, memories, comparators, arithmetic logic units, adders, subtractors, multipliers, dividers, integrators, and any other electronic components configured to perform the operations described in this application. In other examples, one or more of the hardware components that perform the operations described in this application are implemented by computing hardware, for example, by one or more processors or computers. A processor or computer may be implemented by one or more processing elements, such as an array of logic gates, a controller and an arithmetic logic unit, a digital signal processor, a microcomputer, a programmable logic controller, a field-programmable gate array, a programmable logic array, a microprocessor, or any other device or combination of devices that is configured to respond to and execute instructions in a defined manner to achieve a desired result. In one example, a processor or computer includes, or is connected to, one or more memories storing instructions or software that are executed by the processor or computer. Hardware components implemented by a processor or computer may execute instructions or software, such as an operating system (OS) and one or more software applications that run on the OS, to perform the operations described in this application. The hardware components may also access, manipulate, process, create, and store data in response to execution of the instructions or software. For simplicity, the singular term "processor" or "computer" may be used in the description of the examples described in this application, but in other examples multiple processors or computers may be used, or a processor or computer may include multiple processing elements, or multiple types of processing elements, or both. For example, a single hardware component or two or more hardware components may be implemented by a single processor, or two or more processors, or a processor and a controller. One or more hardware components may be implemented by one or more processors, or a processor and a controller, and one or more other hardware components may be implemented by one or more other processors, or another processor and another controller. One or more processors, or a processor and a controller, may implement a single hardware component, or two or more hardware components. A hardware component may have any one or more of different processing configurations, examples of which include a single processor, independent processors, parallel processors, single-instruction single-data (SISD) multiprocessing, single-instruction multiple-data (SIMD) multiprocessing, multiple-instruction single-data (MISD) multiprocessing, and multiple-instruction multiple-data (MIMD) multiprocessing.

The methods that perform the operations described in this application, and illustrated in FIGS. 1-12, are performed by computing hardware, for example, by one or more processors or computers, implemented as described above executing instructions or software to perform the operations described in this application that are performed by the methods. For example, a single operation or two or more operations may be performed by a single processor, or two or more processors, or a processor and a controller. One or more operations may be performed by one or more processors, or a processor and a controller, and one or more other operations may be performed by one or more other processors, or another processor and another controller, e.g., as respective operations of processor implemented methods. One or more processors, or a processor and a controller, may perform a single operation, or two or more operations.

Instructions or software to control computing hardware, for example, one or more processors or computers, to implement the hardware components and perform the methods as described above may be written as computer programs, code segments, instructions or any combination thereof, for individually or collectively instructing or configuring the one or more processors or computers to operate as a machine or special-purpose computer to perform the operations that be performed by the hardware components and the methods as described above. In one example, the instructions or software include machine code that is directly executed by the one or more processors or computers, such as machine code produced by a compiler. In another example, the instructions or software include higher-level code that is executed by the one or more processors or computers using an interpreter. The instructions or software may be written using any programming language based on the block diagrams and the flow charts illustrated in the drawings and the corresponding descriptions in the specification, which disclose algorithms for performing the operations that are performed by the hardware components and the methods as described above.

The instructions or software to control computing hardware, for example, one or more processors or computers, to implement the hardware components and perform the methods as described above, and any associated data, data files, and data structures, may be recorded, stored, or fixed in or on one or more non-transitory computer-readable storage media. Examples of a non-transitory computer-readable storage medium include read-only memory (ROM), random-access programmable read only memory (PROM), EEPROM, RAM, DRAM, SRAM, flash memory, non-volatile memory, CD-ROMs, CD-Rs, CD+Rs, CD-RWs, CD+RWs, DVD-ROMs, DVD-Rs, DVD+Rs, DVD-RWs, DVD+RWs, DVD-RAMs, BD-ROMs, BD-Rs, BD-R LTHs, BD-REs, blue-ray or optical disk storage, hard disk drive (HDD), solid state drive (SSD), flash memory, a card type memory such as multimedia card micro or a card (for example, secure digital (SD) or extreme digital (XD)), magnetic tapes, floppy disks, magneto-optical data storage devices, optical data storage devices, hard disks, solid-state disks, and any other device that is configured to store the instructions or software and any associated data, data files, and data structures in a non-transitory manner and provide the instructions or software and any associated data, data files, and data structures to one or more processors and computers so that the one or more processors and computers can execute the instructions. In one example, the instructions or software and any associated data, data files, and data structures are distributed over network-coupled computer systems so that the instructions and software and any associated data, data files, and data structures are stored, accessed, and executed in a distributed fashion by the one or more processors or computers.

While this disclosure includes specific examples, it will be apparent to one of ordinary skill in the art, after an understanding of the disclosure of this application, that various changes in form and details may be made in these examples without departing from the scope of the claims. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner, and/or replaced or supplemented by other components or their equivalents.

## Claims

1. An apparatus, comprising:
reception nodes configured to receive input signals of an analog domain;
main signal processors configured to perform a signal processing operation on the input signals; and
auxiliary signal processors configured to replace the main signal processors, and perform at least a portion of the signal processing operation in response to a replacement condition being satisfied,
wherein the reception nodes, the main signal processors, and the auxiliary signal processors are implemented in a single integrated circuit (IC) package.

2. The apparatus of claim 1, wherein each of the main signal processors and the auxiliary signal processors comprises an analog digital converter (ADC) performing a digital conversion operation on a corresponding input signal of the input signals.

3. The apparatus of claim 1 or 2, further comprising:
multiplexers (MUXs) configured to provide a signal path used by one of the auxiliary signal processors to replace one of the main signal processors.

4. The apparatus of claim 3, wherein a first main signal processor of the main signal processors receives a first input signal of the input signals from a first reception node of the reception nodes,
a first MUX of the MUXs connects the first reception node to a first auxiliary signal processor of the auxiliary signal processors in response to the replacement condition is satisfied as a defect occurs in the first main signal processor.

5. The apparatus of one of claims 1 to 4, wherein the single IC package comprises at least one die in which the reception nodes, the main signal processors, and the auxiliary signal processors are integrated in at least a partial form of a stack package and a system in package.

6. The apparatus of one of claims 1 to 5, wherein the input signals are received through 10 or more channels, or
wherein the input signals are received through 10,000 or more channels.

7. The apparatus of one of claims 1 to 6, wherein the auxiliary signal processors are configured to perform at least some of the signal processing operation in real-time instead of the main signal processors in response to the replacement condition being satisfied.

8. The apparatus of one of claims 1 to 7, wherein the main signal processors are divided in a predetermined first number and constitute a plurality of slots, and
the auxiliary signal processors are divided in a predetermined second number and dedicatedly and respectively allocated to the plurality of slots.

9. The apparatus of one of claims 1 to 8, wherein the main signal processors are divided in a predetermined first number and constitute a plurality of slots, and
the auxiliary signal processors are allocated to the plurality of slots such that a predetermined third number of slots of the plurality of slots share at least some of the auxiliary signal processors.

10. The apparatus of one of claims 1 to 9, further comprising:
at least one controller configured to determine whether the main signal processors satisfy the replacement condition based on a performance test of the main signal processors.

11. The apparatus of claim 10, wherein the at least one controller is further configured to provide a test signal to the main signal processors while connection between the reception nodes and the main signal processors is open and determine whether the main signal processors satisfy the replacement condition by analyzing each processing result of the main signal processors for the test signal.

12. The apparatus of one of claims 1 to 11, further comprising:
an electrode array measuring the input signal through electrodes disposed on a target point.

13. The apparatus of claim 12, further comprising:
multiplexers (MUXs) connecting candidate electrodes of the electrodes to corresponding auxiliary signal processors of the auxiliary signal processors.

14. The apparatus of claim 13, wherein the main signal processors comprise a first main signal processor connected to a first electrode of the electrodes,
the MUXs comprise a first MUX configured to selectively connect a first auxiliary signal processor of the auxiliary signal processors to first candidate electrodes comprising the first electrode, and
the first MUX is configured to connect the first electrode to the first auxiliary signal processor in response to the replacement condition being satisfied by the first main signal processor.

15. A method comprising:
receiving input signals of an analog domain;
performing a signal processing operation on the input signals using main signal processors; and
performing at least a portion of the signal processing operation using auxiliary signal processors instead of the main signal processors in response to a replacement condition being satisfied,
wherein the main signal processors and the auxiliary signal processors are implemented in a single integrated circuit (IC) package or a single chip.
